# EUROPÄISCHE PATENTANMELDUNG

(11) **EP 0 722 742 A2**
(43) Veröffentlichungstag der Anmeldung: **24.07.1996**
(21) Anmeldenummer: 95119550.2
(22) Anmeldetag: 12.12.1995
(51) Int. Cl.: A61L 9/03, A45D 34/02

(54) **Behälter zur Aufnahme von Wirkstoffen wie Duftstoffen, Insektiziden oder dergleichen**

(30) Priorität: 23.12.1994 DE 4446413
(71) Anmelder: Globol GmbH, D-86633 Neuburg (DE)
(72) Erfinder: Hanauer, Richard, D-86633 Neuburg, Donau (DE); Schimanski, Georg, D-58091 Hagen (DE); Hautmann, Horst, D-86633 Neuburg/Donau (DE)
(74) Vertreter: Reinhard - Skuhra - Weise & Partner

(57) **Zusammenfassung**

In einem Behälter zur Aufnahme von Wirkstoffen wie Duftstoffen, Insektiziden oder dergleichen, insbesondere zum Einsatz in einer Verdampfungsvorrichtung ist ein Docht (3) zum Transport des Wirkstoffes sowie eine erste Kammer (1) zur Aufnahme des Wirkstoffes und eine zweite Kammer (2) vorgesehen. Der Docht (3) von der ersten Kammer (1) erstreckt sich in die zweite Kammer (2), wobei der Docht (3) im wesentlichen flach ausgebildet ist. Eine erste Abdeckung (15) schließt die erste Kammer (1) unter Einschluß des aus der ersten Kammer (1) in die zweite Kammer (2) sich erstreckenden Dochtes (3) ab und die zweite Kammer (2) ist durch eine entfernbare Folie (18) verschlossen.

## Beschreibung

Die Erfindung betrifft einen Behälter zur Aufnahme von Wirkstoffen wie Duftstoffen, Insektiziden oder dgl. gemäß dem Oberbegriff des Patentanspruchs 1.

In der DE 40 11 629 A1 ist eine Vorrichtung zum Verdampfen von Wirkstoffen angegeben, bei der ein in die Vorrichtung einzusetzender Behälter den flüssigen Wirkstoff aufnimmt. Ein in dem Behälter angeordneter Docht transportiert die Wirkstoffe zu einer elektrischen Heizeinrichtung, welche zur Verdampfung der Wirkstoffe beiträgt.

Der Behälter ist mit einem Außengewinde versehen, das zum Einschrauben des Behälters in die Heizvorrichtung dient. Eine derartige Ausbildung von Behältern ist vergleichbar kostenaufwendig. Durch den Behälter selbst wird auch die Komplexität der Vorrichtung maßgeblich beeinflußt. Darüberhinaus muß vor dem Einsetzen des Behälters der diesen normalerweise verschließende Deckel entfernt und beseitigt werden.

Der Erfindung liegt die Aufgabe zugrunde, einen Behälter der eingangs genannten Art anzugeben, der auf einfache Weise geöffnet werden kann und sich auf einfache Weise in eine Verdampfungsvorrichtung einsetzen läßt.

Diese Aufgabe wird erfindungsgemäß durch die im kennzeichnenden Teil des Patentanspruchs 1 angegebenen Merkmale gelöst.

Weitere Ausgestaltungen des Behälters ergeben sich aus den Unteransprüchen.

Die Erfindung schafft einen Behälter zur Aufnahme von Wirkstoffen, welcher sich kostengünstig herstellen läßt und als Nachfüllpackung verfügbar gehalten werden kann. Dieser Behälter läßt sich auf einfache Weise öffnen, bevor er in die zugehörige Vorrichtung zum Verdampfen der Wirkstoffe eingesetzt wird.

Die Handhabung des Behälters vor dem Einsetzen in die zugehörige Vorrichtung ist äußerst einfach, da lediglich eine den Behälter teilweise abschließende Folie abzuziehen ist, um ein Ende eines im Behälter liegenden Dochtes freizugeben. Nach diesem Öffnen des Behälters wird der Behälter in die Vorrichtung eingeschoben und in fester Zuordnung mit dem freien Dochtende gegenüber einer Heizeinrichtung plaziert. Insgesamt läßt sich daher ein derartiges Gerät einfach bedienen. Der Austausch des Behälters nach dem Verbrauch der Wirkstoffe läßt sich schnell und einfach handhaben.

Im folgenden wird ein Behälter zur Aufnahme von Wirkstoffen anhand der Zeichnung zur Erläuterung weiterer Merkmale und Vorteile beschrieben. Es zeigen:
- Fig. 1: eine perspektivische Ansicht einer bevorzugten Ausführungsform eines Behälters;
- Fig. 2 und 3: Ansichten entsprechend Fig. 1 zur Erläuterung der Komponenten des Behälters;
- Fig. 4: eine Vorrichtung zum Verdampfen von Wirkstoffen, wobei Teile weggeschnitten sind;
- Fig. 5: eine schematische Darstellung zur Erläuterung der Plazierung des Behälters in der Vorrichtung nach Fig. 4;
- Fig. 6: eine Perspektivansicht einer bevorzugten Ausführungsform einer Vorrichtung zum Verdampfen von Wirkstoffen entsprechend Fig. 4, und
- Fig. 7: eine Schnittansicht durch eine Vorrichtung zum Verdampfen von Wirkstoffen.

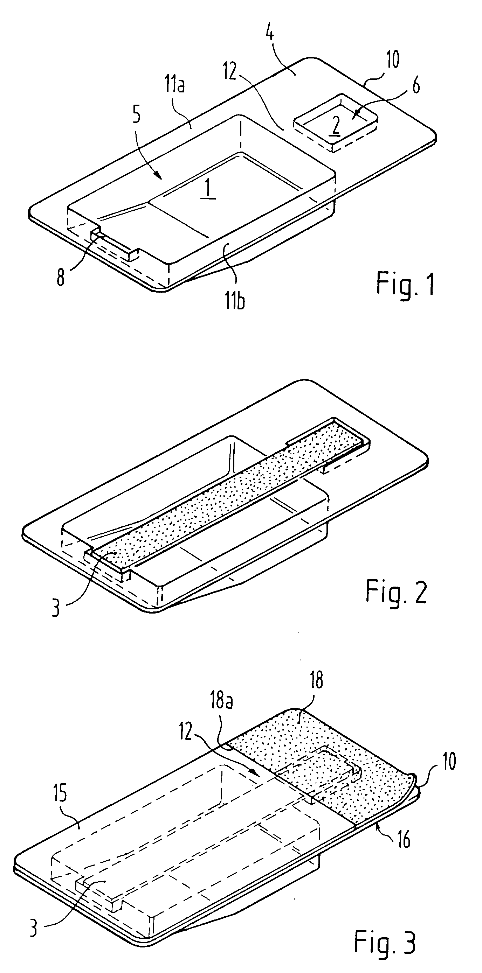

Fig. 1 zeigt eine Perspektivansicht eines Behälters gemäß einer ersten Ausführungsform der Erfindung. Der Behälter besteht vorzugsweise aus Kunststoff, in dem durch Tiefziehen wenigstens zwei Kammern 1, 2 ausgebildet sind. Der Behälter besteht aus einer im wesentlichen rechteckigen Platte aus Kunststoff, in welcher die ebenfalls im wesentlichen rechteckigen Kammern 1, 2 gemäß Fig. 1 nach unten gezogen geformt sind.

Die Kammer 1 dient zur Aufnahme eines Wirkstoffs, während die Kammer 2 im wesentlichen den Zweck hat, ein Ende eines noch nachfolgend unter Bezugnahme auf Fig. 2 beschriebenen Dochtes 3 aufzunehmen.

Die Kammer 2 ist im Verhältnis zur Kammer 1 von wesentlich geringerem Volumen. Zur Festlegung der Kammern 1, 2 sind in der mit 4 bezeichneten Platte oder Fläche aus Kunststoff im wesentlichen rechteckige Öffnungen 5, 6 ausgebildet, von welchen nach unten abstrebende Wandungen die Wandungen und die Böden der Kammern 1, 2 ergeben.

Seitlich der Kammern 1, 2 sind im wesentlichen gerade verlaufende Abschnitte ausgebildet, welche einen vorzugsweise umlaufenden Rand 11a, 11b des Behälters festlegen, der zur Aufnahme von Abdeckungen dient. An dem in Fig. 1 linken bzw. hinteren Ende des Behälters ist eine Aussparung 8 ausgeformt, die geringfügig größere Breite hat als der in den Behälter einzusetzende Docht. Die Aussparung 8 erstreckt sich von der die Kammer 1 festlegenden Öffnung zur benachbarten Kante der Platte 4. Die Kammer 2 ist gegenüber der Kammer 1 um einem vorbestimmten Abstand beabstandet, wie sich aus Fig. 1 ergibt. Die Kammer 2 hält ihrerseits einen Abstand zu einer mit 10 bezeicheten, vorderen Kante des Behälters ein.

Die Perspektivansicht nach Fig. 2 zeigt den Behälter mit eingesetztem Docht 3. Der Docht 3 hat flache Gestalt und eine solche Länge, daß er von der Aussparung 8 des hinteren Behälterteils bis in die Kammer 2 reicht. Obgleich dies in Fig. 2 und 3 nicht gezeigt ist, kann der Docht 3 auch noch eine größere Länge haben, als in Fig. 2 dargestellt, infolgedessen der Docht 3 entweder innerhalb der Kammer 1 bogenförmig nach unten durchgebogen ist und damit von der Aussparung 8 bogenförmig in Richtung auf den Boden der Kammer 1 und dann wieder zurück nach oben verläuft, oder gemäß Fig. 2 gerade verläuft, um aus der Kammer 1 über den zwischen der Kammer 1 und der Kammer 2 definierten Randbereich in die Kammer 2 zu ragen. Der Randbereich zwischen der Kammer 1 und der Kammer 2 ist mit 12 bezeichnet und dient zur Abstützung des Dochtes 3, wobei der Docht 3 im Bereich des Randstreifens 12 praktisch parallel zu diesem Randstreifen 12 und auf dem Randstreifen 12 aufliegend vorgesehen ist. Fig. 2 veranschaulicht den Docht 3 mit im wesentlichen geraden Verlauf, d.h. ohne Durchbiegung im Bereich der Kammer 1.

Wenn der in Fig. 1 und 2 gezeigte Behälter mit dem Wirkstoff gefüllt und der Docht 3 in den Behälter in der in Fig. 2 gezeigten Weise eingesetzt ist, wird zum Verschließen der Kammer 1 eine Kunststoffschicht oder Folie 15 mit vorzugsweise durchsichtiger Eigenschaft so aufgebracht, daß ein vorne verbleibender Abschnitt 16 (Fig. 3) freigehalten wird, wobei der Abschnitt 16 die Kammer 2 beinhaltet. Der sich an die vordere Kante 10 anschließende Abschnitt 16 wird gemäß der Erfindung mit einer Folie 18 aus Metall oder dgl. abgeschlossen, wobei es sich um eine sogenannte "Peel-Off"-Folie handelt. Diese Folie 18 ist mit einer Klebeschicht versehen, die eine feste, luft- und flüssigkeitsdichte Verbindung und damit einen luft- und flüssigkeitsdichten Abschluß gegenüber dem mit 16 bezeichneten Streifen des Behälters sicherstellt. Die Folie 18 muß keine durchsichtige Eigenschaft haben. Damit deckt die Folie 18 die Kammer 2 mit dem darin befindlichen Ende des Dochtes 3 ab. Die mit 18a bezeichnete, nach hinten weisende Kante der Folie 18 grenzt mehr oder weniger unmittelbar an der benachbarten Abdeckung 15 an oder überlappt die Abdeckung 15.

Die Abdeckung 15, die vorzugsweise aus einer Kunststoffschicht oder Kunststoffolie besteht, wird gegenüber dem Behälter im Bereich der nicht weiter definierten Randstreifen durch Thermoversiegelung unlösbar befestigt, während die Folie 18 auf dem Behälter durch Thermoversiegelung entfernbar befestigt ist. Zur Versiegelung der Folie 18 wird vorzugsweise ein Heißsiegellack benutzt, der zwischen der Folie 18 und dem Abschnitt 16 einen Kontakt mit der nach oben weisenden Fläche des Behälters hervorruft. Der Behälter selbst besteht vorzugsweise aus Polyacrylnitril, die Abdeckung 15 aus einem Polyacrylnitril/Polyethylenterephthalat. Die mit 18 bezeichnete Folie wird vorzugsweise aus einem Polyethylenterephthalat/Aluminiumverbund hergestellt und mittels eines Siegellacks auf dem Bereich 16 befestigt.

Aus vorstehender Beschreibung ergibt sich, daß sich der im wesentlichen flache bzw. rechteckige Docht 3 von der Aussparung 8 durch die Kammer 1 über den Steg oder Randstreifen 12 bis in die Kammer 2 erstreckt und dabei insbesondere im Bereich des Steges oder Randstreifens 12 von der Abdeckung 15 gegen den Randstreifen 12 gehalten ist. Der Docht 3 ist damit auch im Bereich des Streifens 12 eingeschlossen und wird durch die Abdeckung 15 in einem vorgegebenen Maße zusammengedrückt, was eine Einschnürung des Dochtes 3 bewirkt und zu einer vorgegebenen oder vorbestimmten Begrenzung des Flüssigkeitstransports aus der Kammer 1 in die Kammer 2 führen kann.

Gemäß der Erfindung ist vorgesehen, daß ein mit Wirkstoff gefüllter Behälter entsprechend Fig. 3, z.B. auch als Nachfüllpackung, bereitgestellt wird und dieser Behälter nach Entfernung der Folie 18 in eine noch zu beschreibende Vorrichtung zur elektrischen Verdampfung des Wirkstoffes eingesetzt wird.

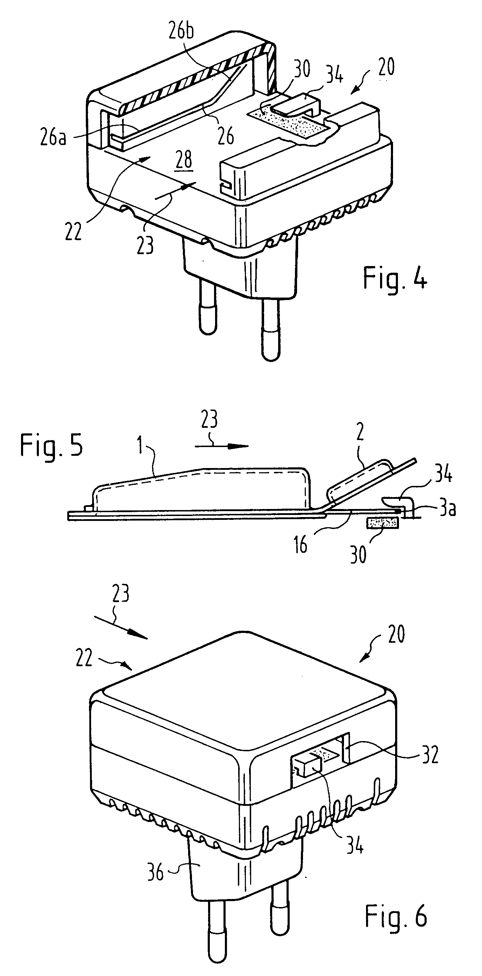

Der bei dem Behälter nach Fig. 1 bis 3 vorgesehene umlaufende Rand 11a, 11b dient teilweise als Führung beim Einsetzen in eine vorzugsweise elektrisch betriebene Vorrichtung zum Verdampfen der Wirkstoffe. Dies wird nachstehend unter Bezugnahme auf Fig. 4 und 5 erläutert.

Die Fig. 4 und 6 zeigen in Teilansicht eine bevorzugte Ausführungsform einer vorzugsweise elektrisch arbeitenden Vorrichtung zum Verdampfen von Wirkstoffen. Gemäß Fig. 4 und 6 ist die dort mit 20 bezeichnete Vorrichtung mit einer Aufnahmekammer 22 versehen, in welche gemäß Fig. 6 in der durch den Pfeil 23 veranschaulichten Richtung ein Behälter der in Verbindung mit den Figuren 1 bis 3 beschriebenen Art eingeführt wird. Die Einführrichtung 23 ergibt sich zudem aus Fig. 5. Zur Führung des Behälters bzw. seiner seitlichen Randstreifen 11a, 11b sind in dem Gehäuse der Vorrichtung nach innen offene Führungsnuten 26 ausgebildet, von welchen in Fig. 4 nur eine Führungsnut 26 veranschaulicht ist. Die Führungsnuten 26 verlaufen parallel zueinander innerhalb jeweils einer Seitenwandung des Gehäuses der Vorrichtung 20 sowie vorzugsweise oberhalb einer im wesentlichen planen Fläche 28, welche die untere Seite bzw. den Boden der Aufnahmekammer 22 festlegt. Anstelle der Führungsnuten 26 können Führungsstege-, schienen-, -nasen oder dgl. vorgesehen sein, welche die gleiche Funktion ausüben. Innerhalb der Fläche 28 ist ein vorzugsweise hinsichtlich seiner Größe begrenztes Gebiet oder Feld 30 vorgesehen, das durch einen Heizwiderstand erhitzt. Das Feld 30 befindet sich am Ende der Aufnahmekammer 22 vor einer Öffnung 32 im Gehäuse der Vorrichtung 20, d.h. am Ende der Aufnahmekammer 22 in Bezug auf die Bewegungsrichtung 23.

Einzelheiten der elektrisch betriebenen Vorrichtung 20 sind nur soweit unter Bezugnahme auf die Fig. 4 und 6 beschrieben und dargestellt, als diese zur Erläuterung der Erfindung erforderlich sind.

Wie Fig. 6 zeigt, ist eine L-förmige Haltelasche bzw. ein Lagerzapfen 34 innerhalb der Aufnahmekammer 22 vor der Öffnung 32 vorgesehen. Die Haltelasche oder Zunge 34 ist am Boden 28 angeformt und erstreckt sich von der Öffnung 32 in die Kammer 22 parallel zum Boden 28. Diese Haltelasche 34 befindet sich vorzugsweise zwischen dem Feld 30 und der Gehäuseöffnung 32 und dient dazu, das im Bereich der Kammer 2 befindliche Ende 3a des Dochtes 3 in vorgegebener Höhe über dem Feld 30 zu halten, sobald der Behälter bis in seine Endposition in die Vorrichtung nach Fig. 4 und 6 eingeschoben ist. Hierdurch wird eine effektive Wärmeübertragung von dem durch einen Heizwiderstand oder dgl. beheizten Feld 30 auf das Ende des Dochtes 3 sichergestellt.

Aus Fig. 5 ergibt sich, daß nach dem Einschieben des Behälters in die Aufnahmekammer 22 die Randstreifen 11a, 11b des Behälters durch die Führungsnuten oder Führungsschlitze 26 der Behälter so geführt wird, daß bei nach oben liegenden Kammern 1, 2 (Fig. 5) und bei abgezogener Verschlußfolie 18 das vorne liegende Ende 3a des Dochtes 3 unter die Haltelasche 34 verlagert ist und dadurch in einem vorbestimmten Abstand zu dem Heizwiderstand bzw. Feld 30 verbleibt, wodurch eine effektive Verdampfung der durch den Docht zu dessen Ende geführten Wirkstoffe ermöglicht wird.

Die Führungsschlitze oder Führungsnuten 26 haben, wie aus Fig. 4 hervorgeht, einen ersten, im wesentlichen gerade verlaufenden Bereich 26a, der im wesentlichen parallel verläuft zur Fläche 28, bevor die Führungsnuten gegenüber der Fläche 28 nach oben geneigt über den mit 26b bezeichneten Bereich innerhalb des Gehäuses der Vorrichtung 20 verlängert sind. In entsprechender Weise zeigt Fig. 5 die durch den Verlauf der Führungsschlitzbereiche 26a, 26b erzwungene Krümmung des Behälters 1, die ein Hochklappen des Abschnitts 16 um eine Schwenkachse im Bereich des Streifens 12 erzwingt. Damit wird das vorne liegende, in Fig. 5 mit 3a bezeichnete Dochtende gegenüber dem Behälterabschnitt 16 ausgelenkt und verläuft im wesentlichen parallel zum hinteren Behälterabschnitt, der die Kammer 1 beinhaltet, während der Bereich 16 mit der Kammer 2 durch den Führungsschlitzbereich 26b in Fig. 5 nach oben angehoben ist, bzw. nur die erwähnte Schwenkachse nach oben (Fig. 5) gekippt bzw. geneigt ist. Durch diese Maßnahme wird der Docht 3 im Bereich seines Endes 3a während des Einschiebens des Behälters in die Aufnahmekammer 22 unter die Haltelasche 34 geführt. In der in Fig. 5 gezeigten Stellung transportiert der Docht 3 aus der Kammer 1 flüssige Wirkstoffe in Richtung auf das Feld 30 zu dem über dem Feld 30 liegenden Dochtabschnitt, wo sie mittels der erzeugten Wärme verdampft und z.B. über die Öffnung 32 aus dem Gehäuse an die Umgebung abgegeben werden.

Die Erfindung schafft einen Behälter zur Aufnahme von Wirkstoffen, insbesondere zum Einsatz in einer Vorrichtung zum Verdunsten der Wirkstoffe. Gemäß der Erfindung weist der Behälter zwei Kammern 1, 2 auf, von denen die eine, größere Kammer mit Wirkstoff gefüllt wird, der über eine durch diese erste Kammer 1 verlaufenden Docht 3 nach dem Einsatz des Behälters in eine Verdunstungsvorrichtung in Richtung auf die zweite Kammer 2 transportiert und dort infolge einer Wärmeeinwirkung intensiv an die Umgebung abgegeben wird. Die Kammer 2, die das durch das Heizgerät zu beaufschlagende Dochtende 3a aufnimmt, wirkt gleichzeitig als Puffer für die Wirkstofflösung, solange der Behälter verschlossen ist, d.h. eventuell über den Docht 3 von der Kammer 1 wegfließende Wirkstoffflüssigkeit wird in der Kammer 2 gespeichert.

Durch die Thermoversiegelung der Kammer 1 und der Kammer 2 wird während der Bereitstellung des Behälters ein luft- und flüssigkeitsdichter Abschluß gewährleistet.

Die den Behälter zum Zwecke der Verdampfung der Wirkstoffe aufnehmende Vorrichtung ist mit Führungsschlitzen 26 versehen, die einen vorbestimmten Abstand zum Boden 28 des Aufnahmeraumes 22 einhalten, wodurch ein sogenannter Kamineffekt bei der Verdampfung der Wirkstoffe über die rückwärtige Gehäuseöffnung 32 der Vorrichtung 20 hinaus gegeben ist.

Die lösbare Abdichtungsfolie 18 läßt sich schnell und einfach von dem Behälter entfernen, gibt dann das nach vorne weisende Dochtende 3a frei, wonach der Behälter, mit den Kammern 1 und 2 nach oben weisend, in die Vorrichtung eingesetzt und bis zum Anschlag durch die Haltelasche 34 eingeschoben werden kann.

Die in Fig. 4 und 6 gezeigte Vorrichtung wird im allgemeinen derart eingesetzt, daß die Öffnung 32 nach oben weist und damit das Dochtende 3a bei vertikal verlaufendem Docht 3 und der in eine Steckdose oder dgl. eingesetzten Vorrichtung 20 gegenüber den Kammern 1, 2 obenliegend angeordnet ist. Bei einer in eine Steckdose eingesetzten Verdünstungsvorrichtung nimmt der Behälter eine Stellung ein, die gegenüber der Darstellung nach Fig. 5 um 90° entgegen dem Uhrzeigersinn gedreht ist.

Die Erfindung schafft einen Behälter zur Aufnahme von Wirkstoffen, sowie eine Vorrichtung, insbesondere elektrische Vorrichtung zum Verdampfen von Wirkstoffen und zur Aufnahme des Behälters, die jeweils als separate Teile ausgebildet sind, wobei der Behälter als Nachfüllbehälter in die Vorrichtung eingesetzt werden kann.

Bei der vorstehend beschriebenen Ausführungsform weist der Behälter im Querschnitt betrachtet im wesentlichen rechteckige Kammern 1, 2 auf. Ersichtlicherweise können die Kammern auch zylindrische oder andere Formen haben, ohne daß die beabsichtigte Funktion beeinträchtigt wird.

Wie sich aus vorstehender Beschreibung ergibt, kann die Vorrichtung zur Aufnahme des beschriebenen Behälters verschiedenartige Führungen 26 aufweisen, welche den Behälter mit seinen Führungsabschnitten 11a, 11b in die in Fig. 5 dargestellte Position innerhalb der Vorrichtung verbringen bzw. dort halten.

Gemäß Fig. 6 ist an der unteren Seite der Vorrichtung zur Verdunstung bzw. Verdampfung der Wirkstoffe ein Steckerteil 36 vorgesehen, welches dazu dient, einen im Feld 30 befindlichen elektrischen Widerstand oder dgl. mit Strom zu beschicken, wodurch die Wärmeerzeugung im Bereich des Feldes 30 hervorgerufen wird, die auf den oberhalb des Feldes 30 liegenden Dochtabschnitt wirkt.

Der Stecker 36 ist gemäß einer bevorzugten Ausführungsform als separates Bauteil konzipiert und läßt sich daher auf unterschiedliche Arten von Vorrichtungen, wie sie in Bezug auf Fig. 4 und 6 dargestellt sind, einsetzen bzw. in eine derartige Vorrichtung einclipsen.

Gemäß einer weiteren Ausgestaltung der Erfindung ist der Stekker 36 um 90° gegenüber dem in Fig. 6 mit 20 bezeichneten Gehäuse, ggf. auch um 360° drehfähig. Die Drehfähigkeit zwischen Stecker 36 und Gehäuse 20 dient insbesondere dazu, die Aufnahmekammer 22 derart auszurichten, daß bei in eine Steckdose eingesetzem Stecker 36 die durch den Pfeil 23 veranschaulichte Richtung stets zur Vertikalen ausgerichtet werden kann.

Gemäß einer bevorzugten Ausführungsform der Erfindung ist vorgesehen, an der Einschiebeöffnung der Vorrichtung nach Fig. 4 eine schwenkbare Klappe vorzusehen, die als Kindersicherung fungiert und die ein unbeabsichtigtes Öffnen und eine unbeabsichtigte Herausnahme des Behälters, z.B. durch Kinder, aus dem Gehäuse verhindern soll. Diese Klappe wird nach Einschieben des Behälters gemäß Fig. 1 bis 3 in die Kammer 22 verschlossen und läßt sich beispielsweise nur mit besonderen Mitteln, z.B. mittels eines Schraubenziehers oder in anderer Weise öffnen, um einen unbefugten Zugang zu dem Behälter auszuschließen.

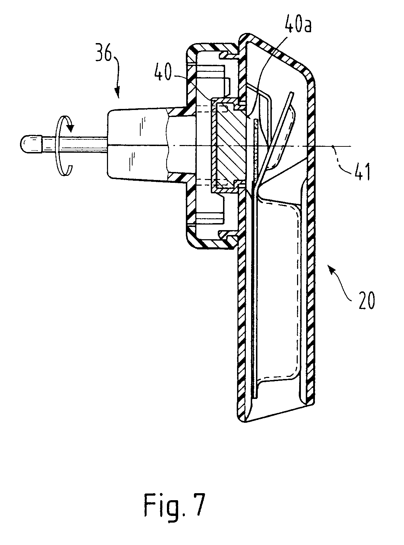

Fig. 7 zeigt eine weitere Abwandlung der Erfindung im Bezug auf die Vorrichtung. Gemäß Fig. 7 ist der Stecker 36 als ein Teil ausgebildet und beinhaltet einen Widerstand 40, der bei dieser Ausführungsform mittig zum Stecker 36 angeordnet ist. Der Stecker 36 zusammen mit dem Widerstand 40 läßt sich um eine mit 41 bezeichnete Achse drehen, wobei der Widerstand 40 zusammen mit dem Stecker 36 um die Achse 41 drehfähig ist. Der Stecker 36 ist am Gehäuse bzw. gegenüber der Vorrichtung 20 drehfähig gelagert. Auf diese Weise läßt sich der Stecker 36 um jeden beliebigen Winkel drehen, je nachdem, in welche Richtung die Vorrichtung 20 ausgerichtet werden soll. Der Widerstand 40 liegt jeweils in Höhe des Feldes 30, um den Docht in der vorstehend beschriebenen Weise mit Wärme zu beaufschlagen.

Fig. 7 zeigt eine Vorrichtung zum Verdunsten von Wirkstoffen oder dergleichen, die ein Gehäuse 20 und einen gegenüber dem Gehäuse 20 drehfähigen Stecker 36 aufweist. Der Stecker 36 mit dem in ihm integriert angeordneten Heizwiderstand 40 ist derart in das Gehäuse 20 eingesetzt, daß eine in Richtung auf das Gehäuse 20 weisende Fläche 40a des Widerstands an einervorbestimmten Stelle des Gehäuses 20 liegt, die einem Element, beispielsweise in Form eines Dochtes, zugeordnet ist, um eine Wirkstoffflüssigkeit, die über diesen Docht zugeführt wird, zu verdampfen. Das Gehäuse 20 weist daher im Bereich des Widerstands 40 bzw. dessen Fläche 40a vorzugsweise eine Öffnung auf, um ein Ende des Dochtes im Bereich der Fläche 40a positionieren zu können. In der Umgebung dieser Öffnung für den Widerstand 40 ist an der zum Stecker 36 weisenden Fläche des Gehäuses 20 eine Führung, beispielsweise in Form einer Nut oder ähnlichem ausgebildet, gegenüber welcher der Stecker 36 drehfähig gelagert ist.

## Patentansprüche

1. Behälter zur Aufnahme von Wirkstoffen wie Duftstoffen, Insektiziden oder dgl., insbesondere zum Einsatz in einer Verdampfungseinrichtung,
mit einem Docht zum Transport des Wirkstoffes,
dadurch **gekennzeichnet**, daß eine erste Kammer (1) zur Aufnahme des Wirkstoffes und eine zweite Kammer (2) vorgesehen sind,
daß sich der Docht (3) von der ersten Kammer (1) in die zweite Kammer (2) erstreckt,
wobei der Docht (3) im wesentlichen flach ausgebildet ist, daß eine erste Abdeckung (15) die erste Kammer (1) unter Einschluß des aus der ersten Kammer (1) in die zweite Kammer (2) sich erstreckenden Dochtes (3) abschließt, und daß die zweite Kammer (2) durch eine entfernbare Folie verschlossen ist.

2. Behälter nach Anspruch 1, dadurch **gekennzeichnet**, daß er aus einer im wesentlichen rechteckigen Platte aus Kunststoff oder dgl. besteht und daß die Kammern (1, 2) durch Tiefziehen gebildet sind.

3. Behälter nach Anspruch 1 oder 2, dadurch **gekennzeichnet**, daß das Volumen der zweiten Kammer (2) im Verhältnis zu dem der ersten Kammer (1) klein ist.

4. Behälter nach wenigstens einem der vorangehenden Ansprüche, dadurch **gekennzeichnet**, daß die Kammern (1, 2) im in der den Behälter festlegenden Platte (4) Öffnungen festlegen.

5. Behälter nach wenigstens einem der vorangehenden Ansprüche, dadurch **gekennzeichnet**, daß die erste Kammer (1) an ihrer zur zweiten Kammer (2) abgewandten Seite eine Aussparung (8) zur Aufnahme eines Endes des Dochtes (3) enthält.

6. Behälter nach wenigstens einem der vorangehenden Ansprüche, dadurch **gekennzeichnet**, daß seitliche Führungsabschnitte (11a, 11b) vorgesehen sind.

7. Behälter nach Anspruch 6, dadurch **gekennzeichnet**, daß die Führungsabschnitte (11a, 11b) in Führungen (26) einer Verdampfungs- bzw. Verdünstungsvorrichtung eingesetzt sind.

8. Behälter nach Anspruch 6, dadurch **gekennzeichnet**, daß die Führungen (26) paarweise und zueinander parallel vorgesehen sind.

9. Behälter nach einem der Ansprüche 6 bis 8, dadurch **gekennzeichnet**, daß jede Führung (26) aus einem Führungsschlitzbereich (26a) besteht, der über eine vorgegebene Länge parallel zu einem Boden (28) einer Aufnahmekammer (22) verläuft, und aus einem zweiten Führungsschlitzabschnitt (26b), der über eine vorbestimmte Länge schräg und nach oben gegenüber dem Boden (28) der Aufnahmekammer (22) gerichtet ist.

10. Behälter nach Anspruch 9, dadurch **gekennzeichnet**, daß die geneigten Führungsschlitzbereiche (26b) über einen Abschnitt der Aufnahmekammer (22) verlaufen, innerhalb welchem eine Heizeinrichtung (30) vorgesehen ist.

11. Vorrichtung zum Verdampfen von Wirkstoffen,
mit einer Heizeinrichtung und mit einem Behälter zur Aufnahme der Wirkstoffe oder dgl., wobei zwischen dem Behälter und der Heizeinrichtung ein Docht angeordnet ist, dadurch **gekennzeichnet**, daß der Behälter eine erste Kammer (1) zur Aufnahme des Wirkstoffes und eine zweite Kammer (2) aufweist, wobei sich der Docht von der ersten Kammer (1) in die zweite Kammer (2) erstreckt, daß der Docht (3) im wesentlichen flach ausgebildet ist, daß eine erste Abdeckung (15) die erste Kammer (1) unter Einschluß des sich aus der ersten Kammer (1) in die zweite Kammer (2) erstreckenden Dochtes (3) abschließt, und daß die zweite Kammer (2) durch eine entfernbare Folie verschlossen ist.

12. Vorrichtung zum Verdampfen von Wirkstoffen, mit einem Gehäuse zur Aufnahme eines Behälters mit Wirkstoffen oder dergleichen, mit einer Heizeinrichtung und einem am Gehäuse angeordneten Stecker,
wobei eine Einrichtung zur Führung der Wirkstoffe zu der Heizeinrichtung vorgesehen ist und der Behälter mit Wirkstoffen in das Gehäuse eingesetzt ist,
**dadurch gekennzeichnet, daß** der Stecker (36) mit der Heizeinrichtung (40) einstückig ausgebildet ist,
daß der Stecker unbegrenzt drehfähig gegenüber dem Gehäuse (20) angeordnet ist, und
daß die Heizeinrichtung (40) eine beheizte Fläche (40a) festlegt, die auf einer zum Gehäuse (20) weisende Seite des Steckers (36) festgelegt ist.

13. Vorrichtung nach Anspruch 11 oder 12, dadurch **gekennzeichnet**, daß Führungen (26a, 26b) zur Aufnahme des Behälters innerhalb einer Aufnahmekammer (22) vorgesehen sind.

14. Vorrichtung nach Anspruch 11, 12 oder 13, dadurch **gekennzeichnet**, daß die Führungen (26a, 26b) aus einem ersten, im wesentlichen parallel zum Boden (28) der Aufnahmekammer (22) verlaufenden und aus einem geneigt zum Boden (28) verlaufenden Abschnitt bestehen.

15. Vorrichtung nach einem der Ansprüche 11 bis 14, dadurch **gekennzeichnet**, daß am Boden (28) der Aufnahmekammer (22) eine Aufnahmezunge (34) ausgebildet ist, die zur Erfassung eines Endes (3a) des Dochtes ausgebildet ist.

16. Vorrichtung nach Anspruch 15, dadurch **gekennzeichnet**, daß im Boden (28) eine Heizeinrichtung (30) vorgesehen ist.

17. Vorrichtung nach Anspruch 15, dadurch **gekennzeichnet**, daß die Heizeinrichtung im wesentlichen unterhalb der Haltelasche (34) ausgebildet ist.

18. Vorrichtung nach einem der Ansprüche 11 bis 17, dadurch **gekennzeichnet**, daß der zur Haltelasche (34) weisende Behälterabschnitt (16) gegenüber dem Boden (28) der Aufnahmekammer (22) geneigt ist.

19. Vorrichtung nach einem der Ansprüche 11 bis 17, **dadurch gekennzeichnet, daß** die Aufnahmekammer (22) durch eine Kindersicherung, beispielsweise in Form einer Verschlußklappe, geschlossen ist.
